# EUROPEAN PATENT SPECIFICATION

(11) **EP 0 438 200 B1**
(45) Date of publication and mention of the grant of the patent: **17.07.2002**
(21) Application number: 91200074.2
(22) Date of filing: 16.01.1991
(51) Int. Cl.: C12N 15/81, C12N 15/16, C12N 15/57, C12N 15/56, C12N 1/16

(54) **Method for the expression of heterologous genes in the yeast Pichia pastoris, expression vectors and transformed microorganisms**
Verfahren zur Expression heterologischer Gene in der Hefe Pichia Pastoris, Expressionsvektoren und transformierte Mikroorganismen
Méthode d'expression de gènes hétérologues dans la levure Pichia pastoris, vecteurs d'expression et microorganismes transformés

(30) Priority: 16.01.1990 CU 790; 13.06.1990 CU 10490; 12.07.1990 CU 13290; 15.08.1990 CU 14290
(43) Date of publication of application: 24.07.1991
(73) Proprietor: CENTRO DE INGENIERIA GENETICA Y BIOTECNOLOGIA, Havana (CU)
(72) Inventor: Herrera Martinez, Luis Saturnino, Playa, Ciudad de la Habana (CU); Yong Gonzalez, Vladimir, Marianao, Ciudad de la Habana (CU); Margollez Clark, Emilio, Maceo, Cerro, Ciudad de la Habana (CU); Delgado Boada, Julio Marcos, Cubanacán, Playa, Ciudad de la Habana (CU); Morales Grillo, Juan, Habana Vieja, Ciudad de la Habana (CU); Torrens Madrazo, Isis del Carmen, Vedado, Plaza de la Revolucion, Habana (CU); Silva Rodriguez, Alejandro, Cubanacán, Playa, Habana (CU); Paifer Reyes, Edenia, Playa, Ciudad de la Habana (CU); Ferbeyre Binelfa, Gerardo, centro Habana, Ciudad de la Habana (CU); S osa Espinosa, Angela Estela, Cubanacán,Playa, Ciudad de la Habana (CU); Martinez Santana, Vladimir, Plaza de la Revolucion, C. de la Habana (CU); Aguiar Santiago, Jorge Agustin, Vista Alegre, Vibora, C. de la Habana (CU); Seralena Menéndez, Alina, Playa, Ciudad de la Habana (CU); Gonzalez Lúpez, Tania, Calle L-Vento, No. 10013, St. Catalina, Cerro, Habana (CU); Montesinos Segui, Raquel, Cabanacán, Playa, Ciudad de la Habana (CU); Cremata Alvarez, José Alberto, Boyeros, Boyeros, Ciudad de la Habana (CU); Villareal Barrios, Adelaida, Cubanacán, Playa, Cuidad de la Habana (CU); González Badillo, Beatriz, Cubanacán, Playa, Ciudad de la Habana (CU); Menéndez Alarcún, Alfredo, Cubanacán, Playa, Ciudad de la Habana (CU)
(74) Representative: Smulders, Theodorus A.H.J., Ir.

(56) References cited:
- EP-A- 0 137 710
- EP-A- 0 261 534
- EP-A- 0 263 311
- EP-A- 0 324 258
- EP-A- 0 329 127
- WO-A-85/00369
- BIOTECHNOLOGY vol. 5, no. 12, December 1987, NEW YORK US pages 1305 - 1308; J.F.Tschopp et al.: "High-level secretion of glycosylated invertase in the methylotrophic yeast, Pichia pastoris"
- GENE. vol. 23, no. 3, September 1983, AMSTERDAM NL pages 267 - 276; Ortlepp et al.: "Molecular cloning in Bacillus subtilis of a Bacillus licheniformis gene encoding a thermostable alpha amylase"
- CHEMICAL ABSTRACTS, vol. 108, 1988 Columbus, Ohio, USA Yamashita Takashi et al.: "Secretion of Mucor rennin, a fungal aspartic protease of Mucor & MGG, Mol. Gen. Genet. vol.210,1987,No.3,pp.462-7 ref. no. 217200Y

## Description

The present invention is in the field of biotechnology and relates to recombinant DNA techniques, in particular to a new method for the efficient expression of heterologous genes in recombinant methylotrophic yeasts of the genus Pichia.

Within this field, an important object has been to develop host-vector systems useful in obtaining desired gene products, the great majority of which cannot be obtained in significant quantities from their natural sources. Numerous prokaryotic and eukaryotic expression systems have been described and used successfully to produce heterologous proteins with high yields.

The cellular organism most exploited for these purposes has been the bacterium E. coli. This is to a large extent due to the knowledge which exists on its gene regulation and to the high feasibility of using it on a large scale.

Nevertheless, E. coli is not the most suitable host for the expression of any heterologous product, especially for proteins of pharmaceutical or dietary interest, since this organism produces toxic and pyrogenic components in its cell wall. Moreover, the mechanisms of transcription, translation and posttranslational processing differ from those of eukaryotic organisms, and hence proteins of eukaryotic origin produced in E. coli may differ from the normal gene products and lack the required biological activity. On the other hand, these products are usually insoluble and form protein aggregates that can be solubilised only by chemical modification, which may lead to a considerable decrease in their specific activity or in an increase in antigenic capacity of the product obtained. Another disadvantage of this host is its limited capacity for excretion of the proteins into the culture medium (E. Baron, "E. coli: an Old Friend but New Industrial Microbe", pp. 27-37; J. Davies, "Retrospect on Heterologous Gene Expression", pp. 17-26. Fifth International Symposium on the Genetics of Industrial Microorganisms, 1986. M. Alacevic, D. Hranueli, Z. Toman, eds.).

The ability to produce heterologous proteins in eukaryotic systems such as yeasts has certain advantages in relation to systems of the prokaryotic type. Among these advantages may be mentioned the ability to grow to high densities and therefore to adapt their cultures to continuous systems (US patent No. 4414329, assigned to Philips Petroleum Co.). Moreover yeasts may excrete the proteins into the culture medium in considerably higher quantities in comparison with E. coli, and the culture media used for yeast fermentation are generally more economical than those which are used in bacteria (Y. Lemoine, "Heterologous Expression in Yeast", 8th International Biotechnology Symposium, Paris, July 17-22, 1988).

Furthermore, these systems can perform posttranslational modifications such as glycosylation which is absent in bacterial systems (W. Fiers, "Engineering Maximal Expression of Heterologous Genes in Microorganisms", 8th International Biotechnology Symposium, Paris, July 17-22, 1988) and moreover may exhibit certain preferences for the codons used by cells of higher organisms, which promotes greater expression of genes from mammals (S.M. Kigsman et al. "Heterologous Gene Expression in Saccharomyces cerevisiae", Biotechnology & Genetic Engineering Reviews, Vol. 3, Ed. G.E. Russell).

In recent years methylotrophic yeasts, and among them in particular Pichia pastoris, have been used successfully for the cloning and expression of heterologous genes (Australian patent application No. 581107 by Philips Petroleum Co.), which makes it an attractive host for a number of advantageous characteristics over the one traditionally used for these purposes, Saccharomyces cerevisiae. The advantages comprise the ability to grow to very high cell densities and to use as the substrate for induction of the cloned genes, a cheap carbon source obtained as a waste material, as is the case with methanol. On the other hand, the system of gene expression of Pichia is more highly regulable compared with the expression systems described for Saccharomyces, which is an advantage for obtaining products which may have harmful effects in the hosts which produce them.

European patent application No. 183070 by Philips Petroleum describes a method for the transformation of a mutant of the yeast Pichia pastoris which is auxotrophic for the enzyme histidinol dehydrogenase, using as a selection marker the HIS4 gene of Saccharomyces cerevisiae and the HIS4 of the actual yeast Pichia pastoris, for which different transformation vectors of the replicative and integrative type were used.

Tschopp et al, Biotechnology 5, 1987, 1305-1308, disclose a method for expressing the sucrose invertase SUC2 gene of Saccharomyces cerevisiae in a his⁻ mutant of Pichia pastoris using an expression vector which comprises the AOX1 promoter of Pichia pastoris, the signal peptide sequence and sucrose invertase coding sequence of the SUC2 gene of Saccharomyces cerevisiae and the HIS4 selection marker of Saccharomyces cerevisiae. Tschopp et al obtain sucrose invertase levels of up to 2.5 g/l and 80-90% of the enzyme is secreted into the growth medium.

The invention provides a method for the expression of heterologous genes in the yeast Pichia pastoris, comprising the steps of preparing an expression vector which contains the heterologous gene to be expressed, transforming a Pichia pastoris host strain, growing a transformant which produces the expression product of the heterologous gene and optionally isolating the expression product formed, wherein the expression vector is selected from
(i) the expression plasmid pPS-7A (CBS 452.90) which contains an expression cassette comprising a fusion of the Pichia pastoris AOX₁ promotor and the signal peptide sequence of the Saccharomyces cerevisiae SUC2 gene, followed by the S. cerevisiae sucrose invertase SUC2 gene, including its own transcription terminator, as a heterologous gene to be expressed, and the S. cerevisiae HIS3 gene as a selection marker, said expression cassette being flanked by 5' and 3' sequences of the Pichia AOX gene to allow integration in the Pichia genome, and
(ii) expression plasmids which contain an expression cassette comprising a fusion of the Pichia pastoris AOX₁ promotor and the signal peptide sequence of the S. cerevisiae SUC2 gene, followed by a heterologous gene to be expressed, the S. cerevisiae GAP gene transcription terminator, and the S. cerevisiae HIS3 gene as a selection marker, said expression cassette being flanked by 5' and 3' sequences of the Pichia AOX gene to allow integration in the Pichia genome,
and wherein an auxotrophic his⁻ mutant of Pichia pastoris which is defective in the enzyme imidazole glycerophosphate dehydratase is used as the host strain.

The invention further provides an expression vector for use with an auxotrophic his⁻ mutant of Pichia pastoris which is defective in the enzyme imidazole glycerophosphate dehydratase, said expression vector being selected from
(i) the expression plasmid pPS-7A (CBS 452.90) which contains an expression cassette comprising a fusion of the Pichia pastoris AOX₁ promotor and the signal peptide sequence of the Saccharomyces cerevisiae SUC2 gene, followed by the S. cerevisiae sucrose invertase SUC2 gene, including its own transcription terminator, as a heterologous gene to be expressed, and the S. cerevisiae HIS3 gene as a selection marker, said expression cassette being flanked by 5' and 3' sequences of the Pichia AOX gene to allow integration in the Pichia genome, and
(ii) expression plasmids which contain an expression cassette comprising a fusion of the Pichia pastoris AOX₁ promotor and the signal peptide sequence of the S. cerevisiae SUC2 gene, followed by a heterologous gene to be expressed, the S. cerevisiae GAP gene transcription terminator, and the S. cerevisiae HIS3 gene as a selection marker, said expression cassette being flanked by 5' and 3' sequences of the Pichia AOX gene to allow integration in the Pichia genome.

Furthermore, the invention provides a Pichia pastoris strain obtained by transformation of an auxotrophic his⁻ mutant of Pichia pastoris which is defective in the enzyme imidazole glycerophosphate dehydratase with an expression vector, said expression vector being selected from
(i) the expression plasmid pPS-7A (CBS 452.90) which contains an expression cassette comprising a fusion of the Pichia pastoris AOX₁ promotor and the signal peptide sequence of the Saccharomyces cerevisiae SUC2 gene, followed by the S. cerevisiae sucrose invertase SUC2 gene, including its own transcription terminator, as a heterologous gene to be expressed, and the S. cerevisiae HIS3 gene as a selection marker, said expression cassette being flanked by 5' and 3' sequences of the Pichia AOX gene to allow integration in the Pichia genome, and
(ii) expression plasmids which contain an expression cassette comprising a fusion of the Pichia pastoris AOX₁ promotor and the signal peptide sequence of the S. cerevisiae SUC2 gene, followed by a heterologous gene to be expressed, the S. cerevisiae GAP gene transcription terminator, and the S. cerevisiae HIS3 gene as a selection marker, said expression cassette being flanked by 5' and 3' sequences of the Pichia ACX gene to allow integration in the Pichia genome.

The present invention provides, and relates to, a method for the expression of heterologous genes which uses as the host a new auxotrophic mutant strain of strain BKM-90 of Pichia pastoris (BKM, Moscow, USSR), which was called MP-36. This mutant is defective in the enzyme imidazole glycerophosphate dehydratase (IGP dehydratase) of the biosynthetic pathway of histidine and was obtained by mutagenesis with N-methyl-N-nitro-nitroso-guanidine (NTG) and with enrichment using nystatin.

A novel feature of the method is the use of the HIS3 gene of S. cerevisiae as a selection marker in the new mutant strain of the genus Pichia.

This mutant was found to display high stability (frequency of reversion 10⁻⁸), as well as to be capable of being transformed with high efficiency, which even surpasses the results expected for vectors of the integrative type such as those which are used in the present invention.

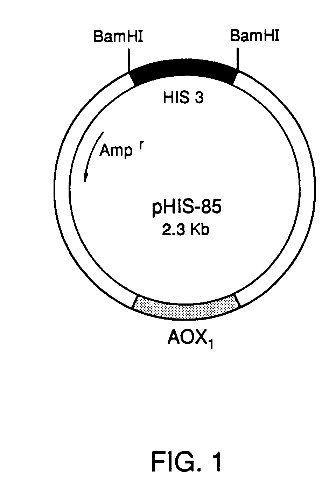

The vector constructed in order to accomplish selection of the mutant MP-36 is pHIS-85 (Fig. 1). This vector contains the HIS3 gene of S. cerevisiae which serves as a selection marker and also contains a chromosomal DNA fragment of Pichia pastoris which serves for integration.

This vector is constructed in order to check that the HIS3 gene of S. cerevisiae can complement a his⁻ mutation of Pichia pastoris and that the mutant MP-36 of said yeast can be transformed under these conditions in a highly efficient manner.

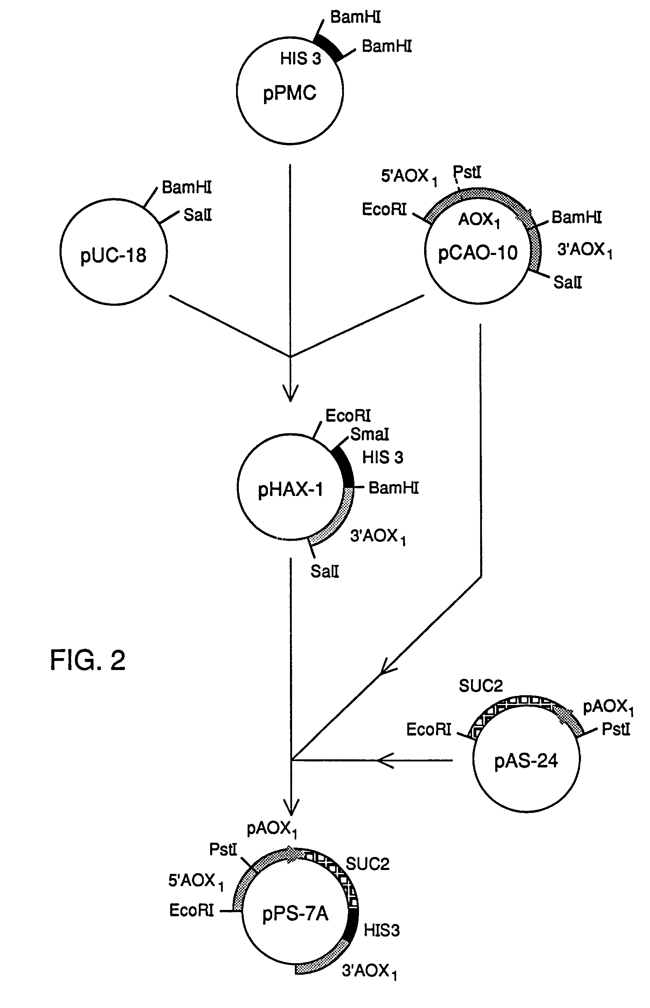

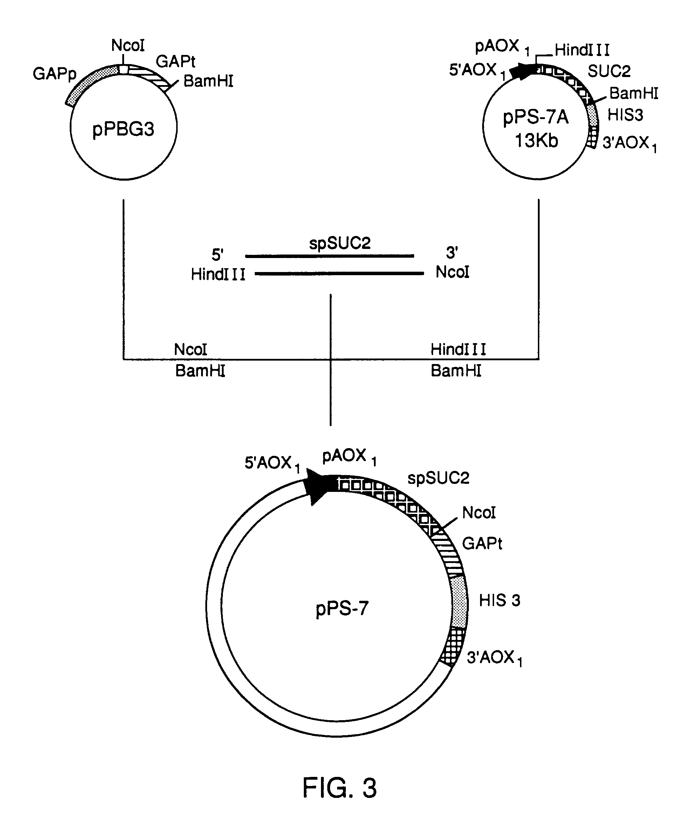

Another novel feature of this method is the use of the integrative vector pPS-7 (Fig. 3) as a cloning vehicle for heterologous genes in Pichia pastoris. A characteristic of this vector is that it contains the HIS3 gene of S. cerevisiae as a selection marker. It also contains the promotor of the alcohol oxidase gene of Pichia pastoris (AOX₁), followed by the signal peptide of the sucrose invertase gene (SUC2) of S. cerevisiae; as a transcription terminator, it contains the transcription terminator of glyceraldehyde 3-phosphate dehydrogenase (GAPt) of S. cerevisiae. Alternatively, the termination signal of the actual gene which it is desired to express can be used (vector pPS-7A, Fig. 2).

The present invention further provides, and relates to, the expression vectors pPEGF-L, pPS-7A, pPSA-3, pPPC316-3 and pRH-4, which are used in a transformation of the mutant MP-36 to obtain heterologous proteins such as epidermal growth factor, invertase, alpha-amylase, bovine rennin and fungal rennin respectively.

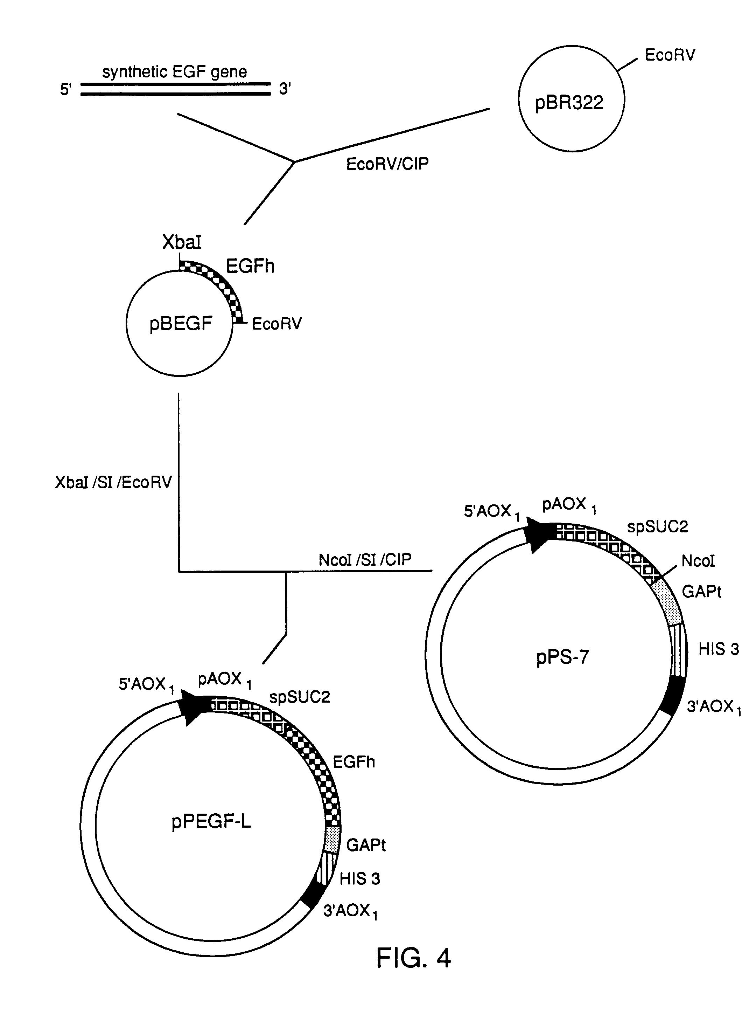

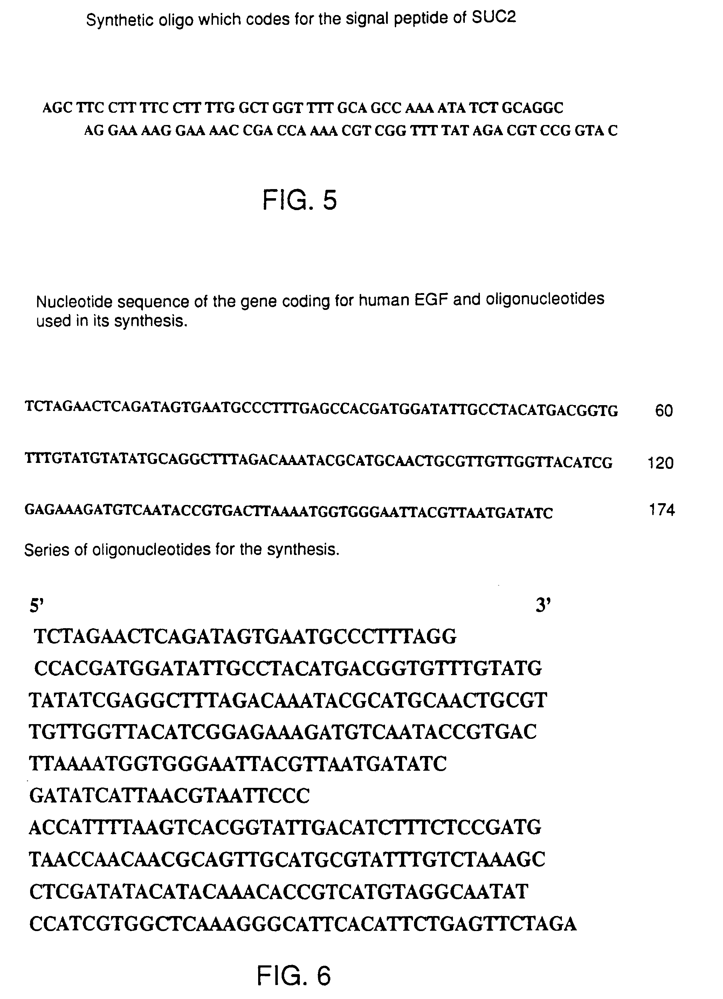

More specifically, for the expression of a heterologous gene there was selected the gene coding for human epidermal growth factor (hEGF) which is obtained by synthesis (Fig. 6). This gene was inserted in the integrative vector pPS-7 between the signal peptide of SUC2 and GAPt (Fig. 4). The host MP-36 was transformed with this vector. The clone MEGF-5, to which the present invention also relates and which, on being fermented under suitable conditions and induced with methanol, displays levels of expression over 2.5 g/l culture, was selected from the transformants.

The high levels of expression reached for this protein made it possible to develop an efficient production process resulting in an end product of high purity which is suitable for pharmaceutical application

For the expression of sucrose invertase there was used the transformation vector pPS-7A, which differs from pPS-7 in that it does not contain GAPt. The host MP-36 was transformed therewith and the clone MSUC-2, to which the present invention also relates, was selected from the transformants. The levels of enzyme expression reached were greater than 4 g/l, the efficiency of this method for the expression of heterologous genes using its own transcription terminator being verified.

The recombinant strain obtained, MSUC-2, has the characteristic of retention of the enzyme in the periplasmic space, which makes it more practical for use in immobilised cell systems.

In the case of alpha-amylase, a transformation of the mutant MP-36 with the vector pPSA-3 resulted in the clone MPA 3625 which is capable of hypersecretion of a thermo-stable alpha-amylase enzyme in quantities greater than 6 g/l culture. The present invention also relates to this clone.

The present invention further provides, and relates to, the recombinant strains:
- CLRB-4, able to produce bovine chymosin within a range of 1-2 g/l culture and resulting from a transformation of the mutant MP-36 with the vector pPPC316-3, and
- CLRH-1, able to produce fungal chymosin (mycorennin) with levels greater than 7 g/l under conditions of semicontinuous culture and resulting from transformation of the mutant MP-36 with the vector pRH-4.

The following strain deposits were made under the rules of the Budapest Treaty with the Centraalbureau voor Schimmel-cultures (CBS), Baarn, the Netherlands, on October 22, 1990:
CBS 454.90, Pichia pastoris CLRB-4 (pPPC316-3)
CBS 453.90, Pichia pastoris CLRH-1 (pRH-4)
CBS 452.90, Pichia pastoris MSUC-2 (pPS-7A)
CBS 451.90, Pichia pastoris MPA 3625 (pPSA-3)
CBS 449.90, Pichia pastoris MEGF-5 (pPEGF-L)

Practical examples of the present invention are described below.

### EXAMPLE 1:

In order to accomplish mutagenesis of Pichia, cultures of yeast strain BKM-90 were inoculated in 100 ml YPG medium (yeast extract 1%, peptone 2%, glucose 2%), and incubated at 30°C for 20 hours. 50 ml of culture were taken and centrifuged at 3000 r.p.m. for 5 min. The cells were washed twice with 0.1 M citrate buffer, pH 5 (sodium citrate 9.79 g, citric acid 3.2 g, and sufficient water for 500 ml) and resuspended in 50 ml of the same buffer. 10 ml of this suspension were taken and treated with a solution of NTG to a final concentration of 50 g/l. The suspension was incubated in the presence of the mutagen for 30 min at 30°C while standing.

The NTG was removed from the suspension by washing twice with distilled water. The cells were resuspended in 50 ml of YPG and transferred to an Erlenmeyer flask with 100 ml of this medium, being incubated at 30°C with agitation for 48 h to allow expression of the mutants. Next 5 ml were extracted to inoculate 100 ml of minimal G0 medium (1.32 g of (NH₄)₂HPO₄; 0.9 g of (NH₄)₂SO₄; 0.50 g of urea in one litre of redistilled water) which were used for enrichment with nystatin. After incubation of this culture until reaching 20 to 30% of the initial optical density (OD), it was treated with 25 ml of a nystatin solution. The treated culture was incubated at 30°C for 30 min without agitation. Subsequently the nystatin was eliminated from the medium by washing the cell suspension twice with distilled water, and resuspending the cells in a volume sufficient to obtain between 150 and 200 colonies per dish.

The colonies were grown in complete medium and replicated in minimal medium (GO) for the isolation of auxotrophic mutants. Then the latter were characterised with respect to their nutritional deficiency, using the plasmid pHIS-85 which carries the HIS3 gene of Saccharomyces cerevisiae (Fig. 1).

Using this method, the mutant MP-36 of Pichia pastoris was isolated, which is defective in the biosynthetic pathway of histidine, and in particular in the enzyme imidazole glycerophosphate dehydratase (IGP dehydratase).

### EXAMPLE 2:

With the object of creating a vector for the expression of heterologous genes in the mutant MP-36, the procedure was as follows:

The vector plasmid pUC-18 was digested with the enzymes BamHI and SalI, being subsequently ligated to the BamHI fragment of the vector pPMC which contains the HIS3 auxotrophy marker of S. cerevisiae; in turn, the BamHI/SalI fragment of the plasmid pCAO-10, which includes the AOX₁ gene plus a 3'-flanking fragment, was ligated. As a result of this triple reaction, the plasmid pHAX-1 was obtained (Fig. 2).

Then the plasmid obtained was digested with the enzymes EcoRI and SmaI and ligated to the EcoRI/PstI fragment of the plasmid pCAO-10, the 5'-flanking fragment of the AOX₁ promotor which is used to increase the frequency of recombination by that end. At the same time there was ligated the EcoRI/PstI fragment of the plasmid pAS-24, previously filled in with the Klenow fragment at the EcoRI end; this region contains the link of the AOX₁ promotor to the coding region of the SUC2 gene. From this reaction was obtained the vector pPS-7A (Fig. 2).

A fragment containing GAPt was extracted from the plasmid pBG3, which contains the GAP operator of S. cerevisiae, by digestion with the enzymes NcoI/BamHI. This fragment was introduced into the plasmid pPS-7A, from which the structural gene of SUC2 was previously extracted by digestion with the enzymes HindIII/BamHI. In this ligation was introduced an 48-mer oligonucleotide with sticky 5' HindIII and 3' NcoI ends (Fig. 5), which restored the signal peptide sequence of the SUC2 gene (spSUC2) which was lost in the digestion, and also restored the recognition site for the enzyme NcoI. The resulting vector plasmid was called pPS-7, and this contained the AOX₁ promotor followed by the signal peptide of SUC2, then GAPt with a NcoI cleavage site between the two in order to allow the insertion of the heterologous gene which it is sought to express; further on the HIS3 gene of S. cerevisiae is found, and finally the 3'-terminal fragment of the AOX gene for integration in the yeast (Fig. 3).

### EXAMPLE 3:

Given the amino acid sequence of human (h) EGF, chemical synthesis of the gene which codes for this protein of approximately 6.2 kDa was performed. This gene has been the subject of prior publication (Chiron Corp., US patent 4783412), as well as chemical synthesis thereof (Earth Chemical, French patent 2566799). By the method described (H. Coster, N.D. Sinha, J. Biernat and J. McManus; NAR, vol. 12, pp. 4539-4557, 1984) there was accomplished the synthesis of a gene of 174 base pairs which codes for hEGF (Fig. 6). Then this gene was inserted in the plasmid pBR322 which had been digested with the restriction enzyme EcoRV and treated with phosphatase (Fig. 4). This ligation resulted in the plasmid pBEGF which was multiplied by transformation of the E. coli strain MC1061. Sufficient plasmid material for extraction of the fragment corresponding to the hEGF gene was purified from this strain. A fragment containing said gene and having blunt ends was successfully extracted by consecutive digestion with the restriction enzymes XbaI, SI and EcoRV.

For expression thereof in yeast, this fragment was introduced into the plasmid pPS-7 (the construction of which is described in the previous example), which was previously digested with the enzymes NcoI, SI and finally treated with phosphatase. The plasmid resulting from this ligation was called pPEGF-L (Fig. 4). Herein the gene coding for hEGF is located after the SUC2 signal peptide, under control of the AOX₁ promotor and combined with the GAP terminator, while the 5' and 3' ends of AOX serve as segments for integration. The transformation in yeast was carried out with this plasmid, and in particular Pichia pastoris his3 mutant MP-36 was used as the host.

The transformed strain, which was called MEGF-5, is capable of producing between 2.5 and 3 g EGF per litre.

### EXAMPLE 4:

For expression of the sucrose invertase gene, P. pastoris strain MP-36 was transformed with the plasmid pPS-7A (obtained in one of the intermediate stages of construction of the vector pPS-7, Fig. 2), which carries the SUC2 gene under control of the AOX₁ promotor, and which has as transcription terminator that of the actual SUC2 gene. A similar experiment was performed using honey as a culture medium (industrial medium), which contained 4% total reducing sugars.

In both cases a high productivity of the system was achieved, between 3.5 and 4 g of enzyme per litre culture medium being obtained, and also having as a distinguishing characteristic the retention of this enzyme in the periplasm of the transformed microorganism.

### EXAMPLE 5:

For cloning of the bovine chymosin gene there was used as a source the construct pCB-125 designed for E. coli (Morales, 1989, Rev. Interferón y Biotecn 5(2)), an NcoI site being formed by the polymerase chain reaction (PCR) technique (R. Saiki, 1985, Science 230: 1350-1354) for subsequent manipulation of the gene, and for this purpose the following oligonucleotides were synthesised chemically: 5' of the gene 5'GTGCCATGGCTGAGATCACCAGGATCCCT 3' (SEQ ID NO:1) 3' of the gene 5'GTGTCAAGATCAGATGGCTTTGGCCAGC 3' (SEQ ID NO:2). After amplification the fragment was digested with NcoI, XbaI and subcloned at these sites in the vector pSAO7 with origin of replication of E. coli, being transformed in strain MC1061 and the transformants being selected by hybridisation of colonies with the 5' oligonucleotide designed for the PCR, and the positive clones being verified by restriction analysis with the enzymes NcoI, XbaI and HindIII.

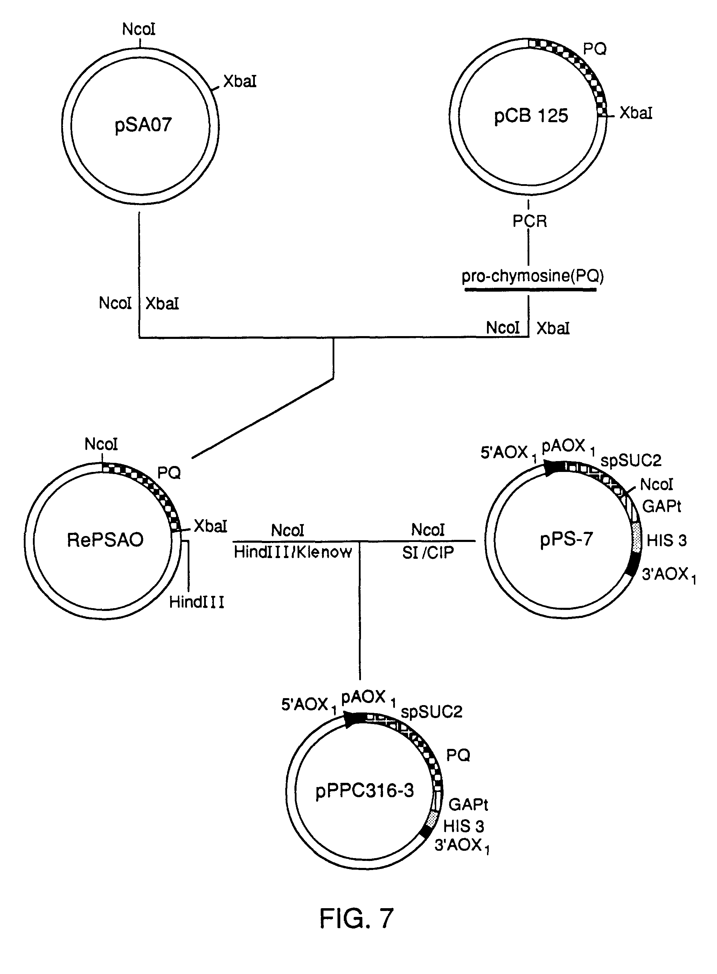

For subcloning of the bovine chymosin gene, the vector pPS7 was linearised with NcoI, and the palindromic ends were made blunt by the action of S1 nuclease, and then phosphorylated. The fragment with the prochymosin gene was obtained from the vector RePSAO by digestion with NcoI, HindIII, and the palindromic ends were filled in, becoming blunt by the action of the Klenow enzyme. In this way it was prepared for introduction thereof in the vector pPS7, giving rise to the integration vector pPPC316-3 (Fig. 7). With this last vector, transformation of the mutant MP-36 was carried out, strain CLRB-4 being obtained which produces chymosin levels greater than 1.5 g/l culture.

### EXAMPLE 6:

For extraction of the gene which codes for aspartic protease of strain IFO 4578 + of Mucor pusillus, purification of the genome of said fungus was carried out. For this, the mycelium was grown in wheat bran extract and the fungal DNA was extracted and purified as reported in the literature (Cryer et al., Methods in Cell Biology, 1975, Vol. XII, pp. 39-44).

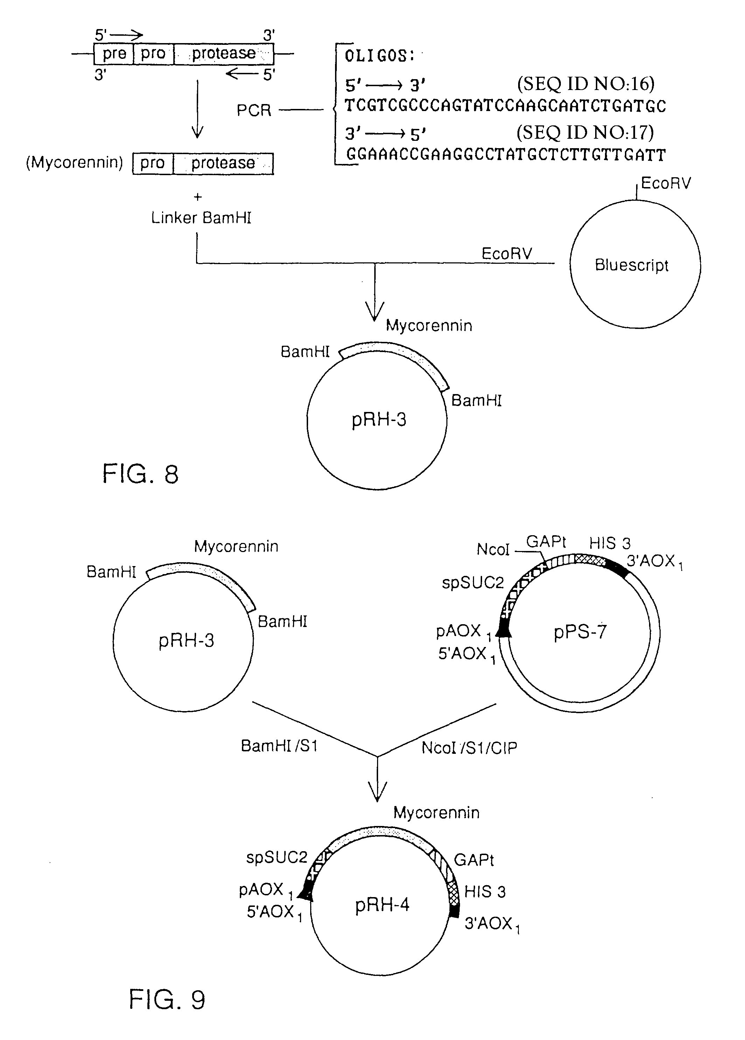

Then the PCR technique was carried out (polymerase chain reaction, R.K. Saiki et al., 1985, Science 230: 1350-1354), for which were used two oligonucleotides the sequences of which are shown in Fig. 8. After amplification and extraction of the gene of the inactive form aspartic proprotease of M. pusillus, this was cloned in Bluescript (G. Wahl, 1989, "Strategies," 2:17), having first been bound to a BamHI linker, to obtain the resulting plasmid pRH-3 (Fig. 8).

In order to carry out cloning of the gene which codes for fungal aspartic protease, the integrative vector pPS7 was cleaved with NcoI, and treated with S1 nuclease and then alkaline phosphatase (CIP) (Maniatis et al., 1982, Molecular Cloning, A Laboratory Manual, Cold Spring Harbor Laboratory, USA). This site permitted introduction of the desired genetic information from the plasmid pRH-3, exactly between the secretion signal of the SUC2 gene of S. cerevisiae and the transcription termination signal of the GAP gene of the Saccharomyces itself, this information remaining under the control of the AOX₁ gene promotor of Pichia pastoris (Fig. 9). This expression cassette was completed with the HIS3 gene of Saccharomyces which was used as a selection marker, and the 5' and 3'-flanking regions of the actual AOX gene which were used for integration. The resulting expression vector was called pRH-4.

Then transformation of Pichia pastoris his- mutant MP36 was carried out with the expression vector pRH-4, and the microorganism resulting from this, called CLRH-1, was grown and induced for excretion of the enzyme under conditions of semicontinuous culture.

For fermentation thereof, the new recombinant strain of the genus Pichia, which produces aspartic protease, was inoculated in a 5-litre fermenter containing industrial medium (13.2 g ammonium phosphate, 9 g ammonium sulphate, 5 g urea and 84 ml molasses per litre of culture medium, which yield 3% total reducing material). The inoculum corresponds to an optical density of 0.5-1 at 600 nm. Fermentation conditions were maintained at an incubation temperature of 30°C and a pH of the medium of 5.5. Induction of the system was carried out by using methanol, when the culture reached the early exponential phase of its growth, and from that moment on a constant flow of 2.5 ml/l/h was maintained.

Under these conditions the recombinant strain CLRH-1 reached excretion levels of the enzyme of the order of 1.5 g/l culture, which increased considerably up to approximately 7 g/l culture by successive replacement with fresh medium as each batch was completed, or by semicontinuous culture.

### EXAMPLE 7:

The chromosomal DNA extracted from Bacillus licheniformis CIB-25 (CIGB's collection of strains) was completely digested with the endonuclease EcoRI, and the fragments having a size between 2 and 4 kb were isolated by low melting point agarose gel electrophoresis (LGT). These fragments were ligated to the vector pBR322 digested with EcoRI and treated with alkaline phosphatase. With the resulting plasmid, E. coli strain MC1066 (F, D lacX74, hsr, hsm, rps 1, galU, galK, trip C 9030F, leuB, pyrF::tn5) was transformed.

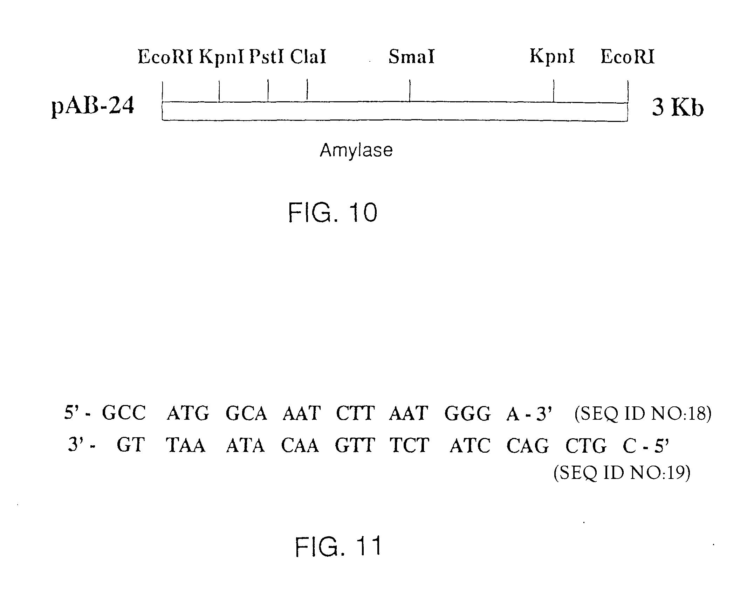

The identification of clones was carried out by activity on plates, by seeding the colonies in LB medium with 0.5% soluble starch and tetracycline at 15 µg/ml. The colonies were left to grow at 37°C for 72 hours and then the plates were stained with metallic iodine vapours. The positive clones were those which displayed formation of a clear halo around the colony. One of the selected clones was called pAB-24, and restriction analysis was carried out with endonucleases. The restriction map of the 3 kb EcoRI fragment which contains the alpha-amylase gene is shown in Fig. 10. The restriction pattern obtained in the central part of the 3 kb fragment coincides with the results of S.A. Ortlepp et al., 1983, Gene 23: 267-276 and G.L. Gray et al., 1986, J. Bacteriol. 166: 635-643.

After analysing the clones and verifying that the structural region coincided with those reported, the region which codes for the gene was isolated with precision. For this purpose 2 synthetic oligonucleotides were designed to perform a polymerase chain reaction (PCR). These oligonucleotides were designed on the basis of the published alpha-amylase sequence of B. licheniformis (G.L. Gray et al., 1986, J. Bacteriol. 166: 635-643). The sequences of both oligonucleotides are shown in Fig. 11. The oligonucleotide corresponding to the 5' region of the gene includes an NcoI site, and the one coresponding to the 3' region of the gene a SalI site. To carry out amplification of the isolated alpha-amylase gene, the method described by R.K. Saiki et al., 1988, Science 239: 487-491, was followed.

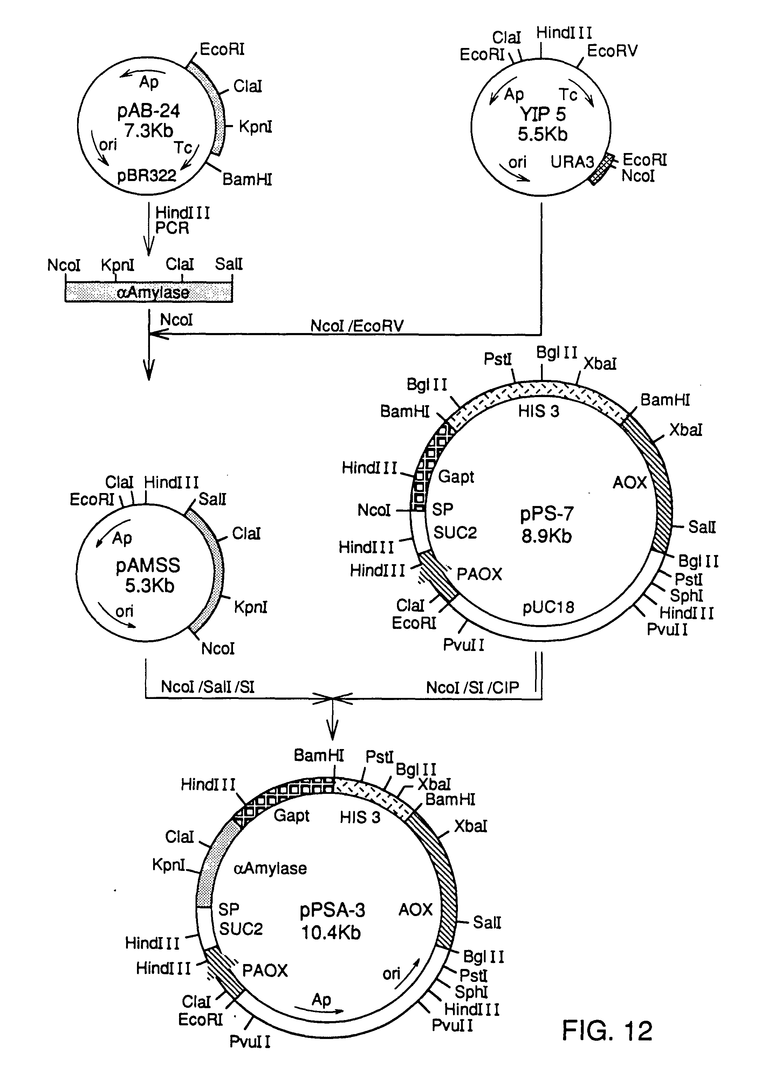

According to the published sequence and the design of the oligonucleotides, the polymerase chain reaction product must yield a 1.4 kb fragment which comprises the mature protein and does not contain its signal sequence. Given that the fragment obtained as the PCR product coincides with the desired size, it is established that there is very high homology between the alpha-amylase gene of B. licheniformis as published and the one isolated in this study. The isolated gene was digested with the enzyme NcoI in order to be subcloned in the plasmid YIP 5, the plasmid pAMSS being formed (Fig. 12).

The plasmid pPS-7 was used as a cloning vector by digestion with the endonuclease NcoI, treatment with SI exonuclease and dephosphorylation of the ends with alkaline phosphatase. In this vector was inserted the alpha-amylase gene obtained from the plasmid pAMSS by digestion with the enzymes NcoI and SalI and making both ends of the fragment blunt with SI exonuclease. In this way they were linked in the final cloning vector pPSA-3 (Fig. 12), which was used for transformation of the mutant MP-36.

The transformants obtained were analysed for their capacity to produce alpha-amylase, for which they were grown on plates of medium G (Galzy and Slonimski, 1957, (Paris) C.R. Acad. Sci., 245: 2423) with 1% soluble starch and 0.5% methanol. After 3 days' incubation at 30°C, the plates were stained with iodine vapours, a clear halo being observed around the yeasts which produce the enzyme, due to starch hydrolysis.

One of the transformed yeasts, MPA 3625, which have a positive result by the plate selection method, was isolated in order to carry out fermentation-induction studies. It was capable of secreting the enzyme alpha-amylase in active form into the culture medium in quantities greater than 6 g/l in a process of fermentation and induction with methanol.

### SEQUENCE LISTING

### SEQ ID NO:1

SEQUENCE TYPE: nucleotide
SEQUENCE LENGTH: 29 nucleotides
MOLECULE TYPE: DNA

GTGCCATGGC TGAGATCACC AGGATCCCT 29

### SEQ ID NO:2

SEQUENCE TYPE: nucleotide
SEQUENCE LENGTH: 28 nucleotides
MOLECULE TYPE: DNA

GTGTCAAGAT CAGATGGCTT TGGCCAGC 28

### SEQ ID NO:3

SEQUENCE TYPE: nucleotide
SEQUENCE LENGTH: 48 nucleotides
MOLECULE TYPE: DNA

AGCTTCCTTT TCCTTTTGGC TGGTTTTGCA GCCAAAATAT CTGCAGGC 48

### SEQ ID NO:4

SEQUENCE TYPE: nucleotide
SEQUENCE LENGTH: 48 nucleotides
MOLECULE TYPE: DNA

CATGGCCTGC AGATATTTTG GCTGCAAAAC CAGCCAAAAG GAAAAGGA 48

### SEQ ID NO:5

SEQUENCE TYPE: nucleotide with corresponding protein
SEQUENCE LENGTH: 174 base pairs
STRANDEDNESS: single
TOPOLOGY: linear
MOLECULE TYPE: genomic DNA
ORIGINAL SOURCE ORGANISM: human
IMMEDIATE EXPERIMENTAL SOURCE: nucleotide synthesis
PROPERTIES: human epidermal growth factor gene

TCTAG AAC TCA GAT AGT GAA TGC CCT TTG AGC CAC GAT GGA TAT 44
Asn Ser Asp Ser Glu Cys Pro Leu Ser His Asp Gly Tyr
1               5                   10
TGC CTA CAT GAC GGT GTT TGT ATG TAT ATC GAG GCT TTA GAC 86
Cys Leu His Asp Gly Val Cys Met Tyr Ile Glu Ala Leu Asp
    15                  20                  25
AAA TAC GCA TGC AAC TGC GTT GTT GGT TAC ATC GGA GAA AGA 128
Lys Tyr Ala Cys Asn Cys Val Val Gly Tyr Ile Gly Glu Arg
        30                  35                  40
TGT CAA TAC CGT GAC TTA AAA TGG TGG GAA TTA CGT TAA TGATATC 174
Cys Gln Tyr Arg Asp Leu Lys Trp Trp Glu Leu Arg *
            45                  50

### SEQ ID NO:6

SEQUENCE TYPE: nucleotide
SEQUENCE LENGTH: 31 nucleotides
MOLECULE TYPE: DNA

TCTAGAACTC AGATAGTGAA TGCCCTTTGA G 31

### SEQ ID NO:7

SEQUENCE TYPE: nucleotide
SEQUENCE LENGTH: 37 nucleotides
MOLECULE TYPE: DNA

CCACGATGGA TATTGCCTAC ATGACGGTGT TTGTATG 37

### SEQ ID NO:8

SEQUENCE TYPE: nucleotide
SEQUENCE LENGTH: 38 nucleotides
MOLECULE TYPE: DNA

TATATCGAGG CTTTAGACAA ATACGCATGC AACTGCGT 38

### SEQ ID NO:9

SEQUENCE TYPE: nucleotide
SEQUENCE LENGTH: 37 nucleotides
MOLECULE TYPE: DNA

TGTTGGTTAC ATCGGAGAAA GATGTCAATA CCGTGAC 37

### SEQ ID NO:10

SEQUENCE TYPE: nucleotide
SEQUENCE LENGTH: 31 nucleotides
MOLECULE TYPE: DNA

TTAAAATGGT GGGAATTACG TTAATGATAT C 31

### SEQ ID NO:11

SEQUENCE TYPE: nucleotide
SEQUENCE LENGTH: 21 nucleotides
MOLECULE TYPE: DNA

GATATCATTA ACGTAATTCC C 21

### SEQ ID NO:12

SEQUENCE TYPE: nucleotide
SEQUENCE LENGTH: 38 nucleotides
MOLECULE TYPE: DNA

ACCATTTTAA GTCACGGTAT TGACATCTTT CTCCGATG 38

### SEQ ID NO:13

SEQUENCE TYPE: nucleotide
SEQUENCE LENGTH: 38 nucleotides
MOLECULE TYPE: DNA

TAACCAACAA CGCAGTTGCA TGCGTATTTG TCTAAAGC 38

### SEQ ID NO:14

SEQUENCE TYPE: nucleotide
SEQUENCE LENGTH: 37 nucleotides
MOLECULE TYPE: DNA

CTCGATATAC ATACAAACAC CGTCATGTAG GCAATAT 37

### SEQ ID NO:15

SEQUENCE TYPE: nucleotide
SEQUENCE LENGTH: 40 nucleotides
MOLECULE TYPE: DNA

CCATCGTGGC TCAAAGGGCA TTCACTATCT GAGTTCTAGA 40

### SEQ ID NO:16

SEQUENCE TYPE: nucleotide
SEQUENCE LENGTH: 30 nucleotides
MOLECULE TYPE: DNA

TCGTCGCCCA GTATCCAAGC AATCTGATGC 30

### SEQ ID NO:17

SEQUENCE TYPE: nucleotide
SEQUENCE LENGTH: 30 nucleotides
MOLECULE TYPE: DNA

TTAGTTGTTC TCGTATCCGG AAGCCAAAGG 30

### SEQ ID NO:18

SEQUENCE TYPE: nucleotide
SEQUENCE LENGTH: 22 nucleotides
MOLECULE TYPE: DNA

GCCATGGCAA ATCTTAATGG GA 22

### SEQ ID NO:19

SEQUENCE TYPE: nucleotide
SEQUENCE LENGTH: 27 nucleotides
MOLECULE TYPE: DNA

CGTCGACCTA TCTTTGAACA TAAATTG 27

## Claims

1. Method for the expression of heterologous genes in the yeast Pichia pastoris, comprising the steps of preparing an expression vector which contains the heterologous gene to be expressed, transforming a Pichia pastoris host strain, growing a transformant which produces the expression product of the heterologous gene and optionally isolating the expression product formed, wherein the expression vector is selected from
(i) the expression plasmid pPS-7A (CBS 452.90) which contains an expression cassette comprising a fusion of the Pichia pastoris AOX₁ promotor and the signal peptide sequence of the Saccharomyces cerevisiae SUC2 gene, followed by the S. cerevisiae sucrose invertase SUC2 gene, including its own transcription terminator, as a heterologous gene to be expressed, and the S. cerevisiae HIS3 gene as a selection marker, said expression cassette being flanked by 5' and 3' sequences of the Pichia AOX gene to allow integration in the Pichia genome, and
(ii) expression plasmids which contain an expression cassette comprising a fusion of the Pichia pastoris AOX₁ promotor and the signal peptide sequence of the S. cerevisiae SUC2 gene, followed by a heterologous gene to be expressed, the S. cerevisiae GAP gene transcription terminator, and the S. cerevisiae HIS3 gene as a selection marker, said expression cassette being flanked by 5' and 3' sequences of the Pichia AOX gene to allow integration in the Pichia genome,
and wherein an auxotrophic his- mutant of Pichia pastoris which is defective in the enzyme imidazole glycerophosphate dehydratase is used as the host strain.

2. Method according to claim 1, wherein the his⁻ auxotrophic Pichia pastoris host strain is transformed with an expression plasmid pPS-7A containing as the heterologous gene to be expressed, the S. cerevisiae sucrose invertase SUC2 gene, including its own transcription terminator, to obtain Pichia pastoris strain MSUC-2 (CBS 452.90).

3. Method according to claim 1, wherein the his⁻ auxotrophic Pichia pastoris host strain is transformed with an expression plasmid pPEGF-L containing as the heterologous gene to be expressed, a human Epidermal Growth Factor gene, and as the transcription terminator, the S. cerevisiae GAP gene transcription terminator, to obtain Pichia pastoris strain MEGF-5 (CBS 449.90).

4. Method according to claim 1, wherein the his⁻ auxotrophic Pichia pastoris host strain is transformed with an expression plasmid pPSA-3 containing as the heterologous gene to be expressed, an alpha-amylase gene, and as the transcription terminator, the S. cerevisiae GAP gene transcription terminator, to obtain Pichia pastoris strain MPA 3625 (CBS 451.90).

5. Method according to claim 1, wherein the his⁻ auxotrophic Pichia pastoris host strain is transformed with an expression plasmid pPPC316-3 containing as the heterologous gene to be expressed, a bovine rennin gene, and as the transcription terminator, the S. cerevisiae GAP gene transcription terminator, to obtain Pichia pastoris strain CLRB-4 (CBS 454.90).

6. Method according to claim 1, wherein the his⁻ auxotrophic Pichia pastoris host strain is transformed with an expression plasmid pRH-4 containing as the heterologous gene to be expressed, a mycorennin gene, and as the transcription terminator, the S. cerevisiae GAP gene transcription terminator, to obtain Pichia pastoris strain CLRH-1 (CBS 453.90).

7. Expression vector for use with an auxotrophic his⁻ mutant of Pichia pastoris which is defective in the enzyme imidazole glycerophosphate dehydratase, said expression vector being selected from
(i) the expression plasmid pPS-7A (CBS 452.90) which contains an expression cassette comprising a fusion of the Pichia pastoris AOX₁ promotor and the signal peptide sequence of the Saccharomyces cerevisiae SUC2 gene, followed by the S. cerevisiae sucrose invertase SUC2 gene, including its own transcription terminator, as a heterologous gene to be expressed, and the S. cerevisiae HIS3 gene as a selection marker, said expression cassette being flanked by 5' and 3' sequences of the Pichia AOX gene to allow integration in the Pichia genome, and
(ii) expression plasmids which contain an expression cassette comprising a fusion of the Pichia pastoris AOX₁ promotor and the signal peptide sequence of the S. cerevisiae SUC2 gene, followed by a heterologous gene to be expressed, the S. cerevisiae GAP gene transcription terminator, and the S. cerevisiae HIS3 gene as a selection marker, said expression cassette being flanked by 5' and 3' sequences of the Pichia AOX gene to allow integration in the Pichia genome.

8. Expression vector according to claim 7, which is pPS-7A (CBS 452.90) containing, as the heterologous gene to be expressed, the S. cerevisiae sucrose invertase SUC2 gene, including its own transcription terminator.

9. Expression vector according to claim 7, which is pPEGF-L (CBS 449.90), pPSA-3 (CBS 451.90), pPPC316-3 (CBS 454.90) or pRH-4 (CBS 453.90) containing, as the heterologous gene to be expressed, a human epidermal growth factor gene, alpha-amylase gene, bovine rennin gene and mycorennin gene, respectively, and as the transcription terminator, the S. cerevisiae GAP gene transcription terminator.

10. Pichia pastoris strain obtained by transformation of an auxotrophic his⁻ mutant of Pichia pastoris which is defective in the enzyme imidazole glycerophosphate dehydratase with an expression vector, said expression vector being selected from
(i) the expression plasmid pPS-7A (CBS 452.90) which contains an expression cassette comprising a fusion of the Pichia pastoris AOX₁ promotor and the signal peptide sequence of the Saccharomyces cerevisiae SUC2 gene, followed by the S. cerevisiae sucrose invertase SUC2 gene, including its own transcription terminator, as a heterologous gene to be expressed, and the S. cerevisiae HIS3 gene as a selection marker, said expression cassette being flanked by 5' and 3' sequences of the Pichia AOX gene to allow integration in the Pichia genome, and
(ii) expression plasmids which contain an expression cassette comprising a fusion of the Pichia pastoris AOX₁ promotor and the signal peptide sequence of the S. cerevisiae SUC2 gene, followed by a heterologous gene to be expressed, the S. cerevisiae GAP gene transcription terminator, and the S. cerevisiae HIS3 gene as a selection marker, said expression cassette being flanked by 5' and 3' sequences of the Pichia AOX gene to allow integration in the Pichia genome.

11. Pichia pastoris strain according to claim 10, which is strain MEGF-5 (CBS 449.90), MSUC-2 (CBS 452.90), MPA 3625 (CBS 451.90), CLRB-4 (CBS 454.90) or CLRH-1 (CBS 453.90), said strain expressing high levels of hEGF, periplasmic sucrose invertase, thermo-stable alpha-amylase, bovine rennin and mycorennin, respectively, resulting from transformation of an auxotrophic his- mutant of Pichia pastoris which is defective in the enzyme imidazole glycerophosphate dehydratase with the expression vector pPEGF-L, pPS-7A, pPSA-3, pPPC316-3 and pRH-4, respectively.

## Patentansprüche

1. Verfahren zur Expression heterologer Gene in der Hefe *Pichia pastoris*, das die folgenden Schritte umfasst: Herstellen eines Expressionsvektors, der das zu exprimierende heterologe Gen enthält, Transformieren eines *Pichia-pastoris*-Wirtsstammes, Züchten einer Transformanten, die das Expressionsprodukt des heterologen Gens erzeugt, und gegebenenfalls Isolieren des gebildeten Expressionsprodukts, wobei der Expressionsvektor ausgewählt ist aus:
(i) dem Expressionsplasmid pPS-7A (CB5 452.90), das eine Expressionscassette enthält, die eine Fusion des *Pichia-pastoris*-AOX₁-Promotors und der Signalpeptidsequenz des *Saccharomycescerevisiae-SUC2-Gens,* gefolgt vom *S.-cerevisiae*-Sucrose-Invertase-SUC2-Gen einschließlich seines eigenen Transcriptionsterminators als zu exprimierendem heterologem Gen und dem *S.-cerevisiae*-HIS3-Gen als Selektionsmarker, umfasst, wobei die Expressionscassette von 5'- und 3'-Sequenzen des *Pichia*-AOX-Gens flankiert ist, um eine Integration in das *Pichia*-Genom zu ermöglichen; und
(ii) Expressionsplasmiden, die eine Expressionscassette enthalten, welche eine Fusion des *Pichia-pastoris*-AOX₁-Promotors und der Signalpeptidsequenz des *S.-cerevisiae*-SUC2-Gens, gefolgt von einem zu exprimierenden heterologen Gen, dem Transcriptionsterminator des *S.-cerevisiae*-GAP-Gens und dem *S.-cerevisiae*-HIS3-Gen als Selektionsmarker, umfasst, wobei die Expressionscassette von 5'- und 3'-Sequenzen des *Pichia*-AOX-Gens flankiert ist, um eine Integration in das *Pichia*-Genom zu ermöglichen;
wobei eine auxotrophe his-negative Mutante von *Pichia pastoris,* der das Enzym Imidazolglycerophosphat-Dehydratase fehlt, als Wirtsstamm verwendet wird.

2. Verfahren gemäß Anspruch 1, wobei der his-negative auxotrophe *Pichia-pastoris*-Wirtsstamm mit einem Expressionsplasmid pPS-7A transformiert wird, das als zu exprimierendes heterologes Gen das *S.-cerevisiae*-Sucrose-Invertase-SUC2-Gen einschließlich seines eigenen Transcriptionsterminators enthält, so dass man den *Pichia-pastoris*-Stamm MSUC-2 (CBS 452.90) erhält.

3. Verfahren gemäß Anspruch 1, wobei der his-negative auxotrophe *Pichia-pastoris*-Wirtsstamm mit einem Expressionsplasmid pPEGF-L transformiert wird, das als zu exprimierendes heterologes Gen ein humanes Epidermiswachstumsfaktor-Gen und als Transcriptionsterminator den Transcriptionsterminator des *S.-cerevisiae*-GAP-Gens enthält, so dass man den *Pichia-pastoris*-Stamm MEGF-5 (CBS 449.90) erhält.

4. Verfahren gemäß Anspruch 1, wobei der his-negative auxotrophe *Pichia-pastoris*-Wirtsstamm mit einem Expressionsplasmid pPSA-3 transformiert wird, das als zu exprimierendes heterologes Gen ein alpha-Amylase-Gen und als Transcriptionsterminator den Transcriptionsterminator des *S.-cerevisiae*-GAP-Gens enthält, so dass man den *Pichia-pastoris*-Stamm MPA 3625 (CBS 451.90) erhält.

5. Verfahren gemäß Anspruch 1, wobei der his-negative auxotrophe *Pichia-pastoris*-Wirtsstamm mit einem Expressionsplasmid pPPC316-3 transformiert wird, das als zu exprimierendes heterologes Gen ein bovines Rennin-Gen und als Transcriptionsterminator den Transcriptionsterminator des *S.-cerevisiae-GAP-Gens* enthält, so dass man den *Pichia-pastoris*-Stamm CLRB-4 (CBS 454.90) erhält.

6. Verfahren gemäß Anspruch 1, wobei der his-negative auxotrophe *Pichia-pastoris*-Wirtsstamm mit einem Expressionsplasmid pRH-4 transformiert wird, das als zu exprimierendes heterologes Gen ein Mycorennin-Gen und als Transcriptionsterminator den Transcriptionsterminator des *S.-cerevisiae*-GAP-Gens enthält, so dass man den *Pichia-pastoris*-Stamm CLRH-1 (CBS 453.90) erhält.

7. Expressionsvektor zur Verwendung mit einer auxotrophen his-negativen Mutante von *Pichia pastoris,* der das Enzym Imidazolglycerophosphat-Dehydratase fehlt, wobei der Expressionsvektor ausgewählt ist aus:
(i) dem Expressionsplasmid pPS-7A (CBS 452.90), das eine Expressionscassette enthält, die eine Fusion des *Pichia-pastoris*-AOX₁-Promotors und der Signalpeptidsequenz des *Saccharomycescerevisiae*-SUC2-Gens, gefolgt vom *S.-cerevisiae*-Sucrose-Invertase-SUC2-Gen einschließlich seines eigenen Transcriptionsterminators als zu exprimierendem heterologem Gen und dem *S.-cerevisiae*-HIS3-Gen als Selektionsmarker, umfasst, wobei die Expressionscassette von 5'- und 3'-Sequenzen des *Pichia*-AOX-Gens flankiert ist, um eine Integration in das *Pichia*-Genom zu ermöglichen; und
(ii) Expressionsplasmiden, die eine Expressionscassette enthalten, welche eine Fusion des *Pichia-pastoris*-AOX₁-Promotors und der Signalpeptidsequenz des *S.-cerevisiae-SUC2-Gens,* gefolgt von einem zu exprimierenden heterologen Gen, dem Transcriptionsterminator des *S.-cerevisiae*-GAP-Gens und dem *S.-cerevisiae*-HIS3-Gen als Selektionsmarker, umfasst, wobei die Expressionscassette von 5'- und 3'-Sequenzen des *Pichia*-AOX-Gens flankiert ist, um eine Integration in das *Pichia*-Genom zu ermöglichen.

8. Expressionsvektor gemäß Anspruch 7, bei dem es sich um pPS-7A (CBS 452.90) handelt, der als zu exprimierendes heterologes Gen das *S.-cerevisiae*-Sucrose-Invertase-SUC2-Gen einschließlich seines eigenen Transcriptionsterminators enthält.

9. Expressionsvektor gemäß Anspruch 7, bei dem es sich um pPEGF-L (CBS 449,90), pPSA-3 (CBS 451.90), pPPC316-3 (CBS 454.90) oder pRH-4 (CBS 453.90) handelt, das als zu exprimierendes heterologes Gen ein humanes Epidermiswachstumsfaktor-Gen, ein alpha-Amylase-Gen, ein bovines Rennin-Gen bzw. ein Mycorennin-Gen und als Transcriptionsterminator den Transcriptionsterminator des *S.-cerevisiae*-GAP-Gens enthält.

10. *Pichia-pastoris*-Stamm, der durch Transformation einer auxotrophen his-negativen Mutante von *Pichia pastoris,* der das Enzym Imidazolglycerophosphat-Dehydratase fehlt, mit einem Expressionsvektor erhalten wird,
wobei der Expressionsvektor ausgewählt ist aus:
(i) dem Expressionsplasmid pPS-7A (CBS 452.90), das eine Expressionscassette enthält, die eine Fusion des *Pichia-pastoris*-AOX₁-Promotors und der Signalpeptidsequenz des *Saccharomyces-cerevisiae*-SUC2-Gens, gefolgt vom *S.-cerevisiae*-Sucrose-Invertase-SUC2-Gen einschließlich seines eigenen Transcriptionsterminators als zu exprimierendem heterologem Gen und dem *S.-cerevisiae*-HIS3-Gen als Selektionsmarker, umfasst, wobei die Expressionscassette von 5'- und 3'-Sequenzen des *Pichia*-AOX-Gens flankiert ist, um eine Integration in das *Pichia*-Genom zu ermöglichen; und
(ii) Expressionsplasmiden, die eine Expressionscassette enthalten, welche eine Fusion des *Pichia-pastoris*-AOX₁-Promotors und der Signalpeptidsequenz des *S.-cerevisiae*-SUC2-Gens, gefolgt von einem zu exprimierenden heterologen Gen, dem Transcriptionsterminator des *S.-cerevisiae*-GAP-Gens und dem *S.-cerevisiae*-HIS3-Gen als Selektionsmarker, umfasst, wobei die Expressionscassette von 5'- und 3'-Sequenzen des *Pichia*-AOX-Gens flankiert ist, um eine Integration in das *Pichia*-Genom zu ermöglichen.

11. , *Pichia-pastoris*-Stamm gemäß Anspruch 10, bei dem es sich um den Stamm MEGF-5 (CBS 449.90), MSUC-2 (CBS 452.90), MPA 3625 (CBS 451.90), CLRB-4 (CBS 454.90) oder CLRH-1 (CBS 453.90) handelt, wobei der Stamm große Mengen von hEGF, periplasmatischer Sucrose-Invertase, thermostabiler alpha-Amylase, bovinem Rennin bzw. Mycorennin exprimiert und durch Transformation einer auxotrophen his-negativen Mutante von *Pichia pastoris*, der das Enzym Imidazolglycerophosphat-Dehydratase fehlt, mit dem Expressionsvektor pPEGF-L, pPS-7A, pPSA-3, pPPC316-3 bzw. pRH-4 erhalten wird.

## Revendications

1. Procédé d'expression de gènes hétérologues chez la levure *Pichia pastoris*, lequel procédé comporte les étapes consistant à préparer un vecteur d'expression contenant le gène à exprimer, à transformer une souche hôte de *Pichia pastoris*, à faire croître une souche transformée qui fabrique le produit de l'expression dudit gène hétérologue, et à isoler, si on le veut, le produit d'expression fabriqué, ledit vecteur d'expression étant choisi parmi :
a) le plasmide d'expression pPS-7A (CBS 452.90), qui contient une cassette d'expression comprenant le promoteur AOX₁ de *Pichia pastoris* fusionné avec la séquence "peptide signal" du gène SUC2 de *Saccharomyces cerevisiae*, puis le gène SUC2 de saccharose invertase de *S. cerevisiae*, y compris son propre signal de fin de transcription, en tant que gène hétérologue à exprimer, et le gène HIS3 de *S. Cerevisiae*, en tant que gène marqueur de sélection, cette cassette d'expression étant flanquée des séquences 5' et 3 ' du gène AOX de *Pichia* qui en permettent l'intégration au génome de *Pichia* ; et
b) les plasmides d'expression qui contiennent une cassette d'expression comprenant le promoteur AOX₁ de *Pichia pastoris* fusionné avec la séquence "peptide signal" du gène SUC2 de *Saccharomyces cerevisiae*, puis un gène hétérologue à exprimer, le signal de fin de transcription du gène GAP de *S. cerevisiae*, et le gène HIS3 de *S. Cerevisiae*, en tant que gène marqueur de sélection, cette cassette d'expression étant flanquée des séquences 5' et 3 ' du gène AOX de *Pichia* qui en permettent l'intégration au génome de *Pichia ;*
et dans lequel procédé l'on emploie pour souche hôte une souche mutante auxotrophe His⁻ de *Pichia pastoris*, déficiente pour ce qui est de l'enzyme imidazole-glycérophosphate déshydratase.

2. Procédé conforme à la revendication 1, dans lequel on transforme la souche auxotrophe His⁻ de *Pichia pastoris* servant de souche hôte avec un plasmide d'expression pPS-7A qui contient, en tant que gène hétérologue à exprimer, le gène SUC2 de saccharose invertase de *S. cerevisiae*, y compris son propre signal de fin de transcription, pour obtenir la souche MSUC-2 de *Pichia pastoris* (CBS 452.90).

3. Procédé conforme à la revendication 1, dans lequel on transforme la souche auxotrophe His⁻ de *Pichia pastoris* servant de souche hôte avec un plasmide d'expression pPEGF-L qui contient, en tant que gène hétérologue à exprimer, un gène d'EGF (facteur de croissance épidermique) humain et, en tant que signal de fin de transcription, le signal de fin de transcription du gène GAP de *S. cerevisiae*, pour obtenir la souche MEGF-5 de *Pichia pastoris* (CBS 449.90).

4. Procédé conforme à la revendication 1, dans lequel on transforme la souche auxotrophe His⁻ de *Pichia pastoris* servant de souche hôte avec un plasmide d'expression pPSA-3 qui contient, en tant que gène hétérologue à exprimer, un gène d'alpha-amylase et, en tant que signal de fin de transcription, le signal de fin de transcription du gène GAP de *S. cerevisiae*, pour obtenir la souche MPA 3625 de *Pichia pastoris* (CBS 451.90).

5. Procédé conforme à la revendication 1, dans lequel on transforme la souche auxotrophe His⁻ de *Pichia pastoris* servant de souche hôte avec un plasmide d'expression pPPC316-3 qui contient, en tant que gène hétérologue à exprimer, un gène de rénine bovine et, en tant que signal de fin de transcription, le signal de fin de transcription du gène GAP de *S. cerevisiae*, pour obtenir la souche CLRB-4 de *Pichia pastoris* (CBS 454.90).

6. Procédé conforme à la revendication 1, dans lequel on transforme la souche auxotrophe His⁻ de *Pichia pastoris* servant de souche hôte avec un plasmide d'expression pRH-4 qui contient, en tant que gène hétérologue à exprimer, un gène de mycorénine et, en tant que signal de fin de transcription, le signal de fin de transcription du gène GAP de *S. cerevisiae*, pour obtenir la souche CLRH-1 de *Pichia pastoris* (CBS 453.90).

7. Vecteur d'expression destiné à être utilisé avec une souche mutante auxotrophe His⁻ de *Pichia pastoris*, déficiente pour ce qui est de l'enzyme imidazole-glycérophosphate déshydratase, ledit vecteur d'expression étant choisi parmi :
a) le plasmide d'expression pPS-7A (CBS 452.90), qui contient une cassette d'expression comprenant le promoteur AOX₁ de *Pichia pastoris* fusionné avec la séquence "peptide signal" du gène SUC2 de *Saccharomyces cerevisiae*, puis le gène SUC2 de saccharose invertase . de *S. cerevisiae*, y compris son propre signal de fin de transcription, en tant que gène hétérologue à exprimer, et le gène HIS3 de *S. Cerevisiae*, en tant que gène marqueur de sélection, cette cassette d'expression étant flanquée des séquences 5' et 3 ' du gène AOX de *Pichia* qui en permettent l'intégration au génome de *Pichia* ; et
b) les plasmides d'expression qui contiennent une cassette d'expression comprenant le promoteur AOX₁ de *Pichia pastoris* fusionné avec la séquence "peptide signal" du gène SUC2 de *Saccharomyces cerevisiae*, puis un gène hétérologue à exprimer, le signal de fin de transcription du gène GAP de *S. cerevisiae*, et le gène HIS3 de *S. Cerevisiae,* en tant que gène marqueur de sélection, cette cassette d'expression étant flanquée des séquences 5' et 3 ' du gène AOX de *Pichia* qui en permettent l'intégration au génome de *Pichia*.

8. Vecteur d'expression conforme à la revendication 7, qui est le plasmide pPS-7A (CBS 452.90) qui contient, en tant que gène hétérologue à exprimer, le gène SUC2 de saccharose invertase de *S. cerevisiae*, y compris son propre signal de fin de transcription.

9. Vecteur d'expression conforme à la revendication 7, qui est l'un des plasmides pPEGF-L (CBS 449.90), pPSA-3 (CBS 451.90), pPPC316-3 (CBS 454.90) et pRH-4 (CBS 453.90), lesquels contiennent respectivement, en tant que gène hétérologue à exprimer, un gène d'EGF (facteur de croissance épidermique) humain, un gène d'alpha-amylase, un gène de rénine bovine ou un gène de mycorénine, ainsi que, en tant que signal de fin de transcription, le signal de fin de transcription du gène GAP de *S. cerevisiae.*

10. Souche de *Pichia pastoris* obtenue par transformation, au moyen d'un vecteur d'expression, d'une souche mutante auxotrophe His⁻ de *Pichia pastoris*, déficiente pour ce qui est de l'enzyme imidazole-glycérophosphate déshydratase, ledit vecteur d'expression étant choisi parmi :
a) le plasmide d'expression pPS-7A (CBS 452.90), qui contient une cassette d'expression comprenant le promoteur AOX₁ de *Pichia pastoris* fusionné avec la séquence "peptide signal" du gène SUC2 de *Saccharomyces cerevisiae*, puis le gène SUC2 de saccharose invertase de *S. cerevisiae*, *y* compris son propre signal de fin de transcription, en tant que gène hétérologue à exprimer, et le gène HIS3 de *S. Cerevisiae*, en tant que gène marqueur de sélection, cette cassette d'expression étant flanquée des séquences 5' et 3 ' du gène AOX de *Pichia* qui en permettent l'intégration au génome de *Pichia* ; et
b) les plasmides d'expression qui contiennent une cassette d'expression comprenant le promoteur AOX₁ de *Pichia pastoris* fusionné avec la séquence "peptide signal" du gène SUC2 de *Saccharomyces cerevisiae*, puis un gène hétérologue à exprimer, le signal de fin de transcription du gène GAP de *S. cerevisiae*, et le gène HIS3 de *S. Cerevisiae*, en tant que gène marqueur de sélection, cette cassette d'expression étant flanquée des séquences 5' et 3 ' du gène AOX de *Pichia* qui en permettent l'intégration au génome de *Pichia.*

11. Souche de *Pichia pastoris* conforme à la revendication 10, qui est la souche MEGF-5 (CBS 449.90), MSUC-2 (CBS 452.90), MPA 3625 (CBS 451.90), CLRB-4 (CBS 454.90) ou CLRH-1 (CBS 453.90), qui produit en grandes quantités, respectivement, de l'EGF humain, une saccharose invertase périplasmique, une alpha-amylase thermostable, une rénine bovine ou une mycorénine, et qui résulte de la transformation d'une souche mutante auxotrophe His⁻ de *Pichia pastoris*, déficiente pour ce qui est de l'enzyme imidazole-glycérophosphate déshydratase, avec un vecteur d'expression qui est, respectivement, pPEGF-L, pPS-7A, pPSA-3, pPPC316-3 ou pRH-4.
